(19) Europäisches Patentamt
European Patent Office
Office européen des brevets

(11) Publication number: 0 488 041 A2

# (12) EUROPEAN PATENT APPLICATION

(21) Application number: 91119810.9

(51) Int. Cl.[5]: A61K 37/02

(22) Date of filing: 21.11.91

(30) Priority: 26.11.90 JP 323518/90

(43) Date of publication of application:
03.06.92 Bulletin 92/23

(84) Designated Contracting States:
AT BE CH DE DK ES FR GB GR IT LI LU NL SE

(71) Applicant: FUJISAWA PHARMACEUTICAL CO., LTD.
4-7, Doshomachi 3-chome Chuo-ku
Osaka-shi Osaka 541(JP)

(72) Inventor: Yokota, Yoshiko
25-8, Wakazono-cho
Ibaraki-shi, Osaka 567(JP)
Inventor: Wakai, Yoshimi
3-14-21, Shonai Sakaemachi
Toyonaka-shi, Osaka 560(JP)

(74) Representative: Türk, Gille, Hrabal
Brucknerstrasse 20
W-4000 Düsseldorf 13(DE)

## (54) A pharmaceutical composition for cytomegalovirus infection.

(57) Use of a compound of the formula

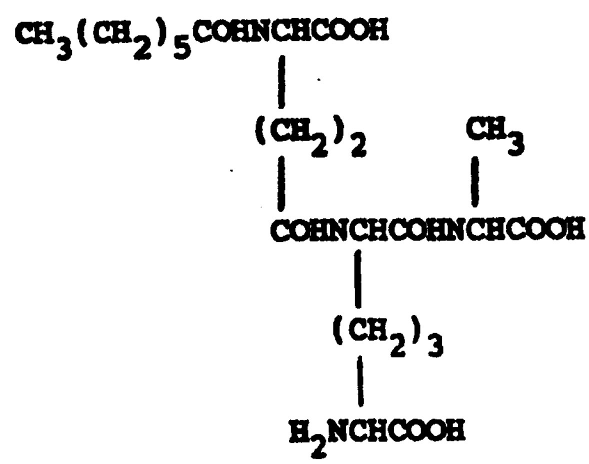

or a pharmaceutically acceptable salt thereof for the preparation of a medicament for preventing or treating infectious diseases due to Cytomegalovirus.

EP 0 488 041 A2

This invention relates to a novel pharmaceutical composition for infectious diseases due to Cytomegalovirus and use of a compound of the formula

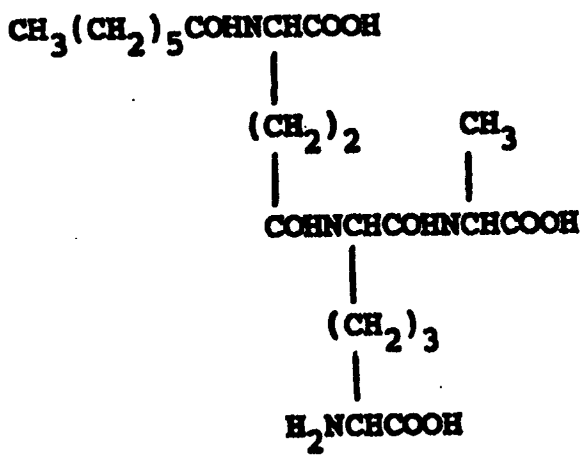

or a pharmaceutically acceptable salt thereof for the preparation of a medicament for preventing or treating infectious diseases due to Cytomegalovirus.

Cytomegalovirus causes serious neonatal infections or iatrogenic infections in the state of compromised immunity. Heretofore, acyclovir is known as a drug for Cytomegalovirus infection but the advent of a more potent drug has been awaited.

Endeavoring to solve the above problems, the inventors of this invention found that a compound of the following formula (I), which is known as a drug for viral infections (cf. U.S. Patent No. 4,666,889), shows specifically potent efficacy in Cytomegalovirus infection and does so more prominently than any of the drugs so far known.

The pharmaceutical composition of this invention for Cytomegalovirus infection contains a compound of the following formula (I) or a pharmaceutically acceptable salt thereof as an active ingredient.

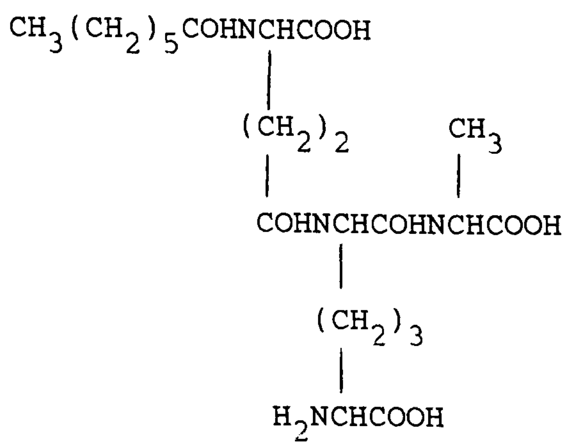

(I)

The pharmaceutical composition of this invention for Cytomegalovirus infection can be used in the prevention and treatment of Cytomegalovirus infection in man and animals.

The pharmaceutically acceptable salt of said active ingredient compound (I) includes, among others, organic or inorganic salts such as sodium salt, potassium salt, calcium salt, ammonium salt, ethanolamine salt, triethylamine salt, dicyclohexylamine salt, etc., and organic or inorganic acid addition salts such as acetate, trifluoroacetate, lactate, maleate, fumarate, tartarate, citrate, methanesulfonate, hydrochloride, sulfate, nitrate, phosphate and so on.

The compound (I) includes one or more stereo-isomers due to the asymmetric carbon within the molecule and any of such isomers can be used as the active ingredient in the pharmaceutical composition of this invention.

The following compound is representative of the compound (I) and pharmaceutically acceptable salt which can be used as the active ingredient in this invention. Compound (A):

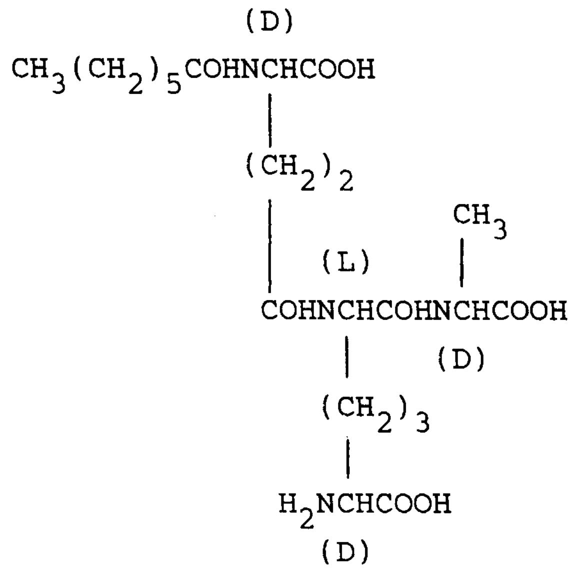

The following test example is intended to illustrate the effect of this invention.

Test Example

Male ICR mice (3 weeks old; 10 per group) were intraperitoneally infected with 2.9 x $10^5$ pfu/mouse of Cytomegalovirus. A predetermined amount of compound (A) (dissolved in water) was subcutaneously administered once daily on day 1 before infection and days 1 and 3 after infection. As a control, the commercial drug acyclovir was subcutaneously administered twice daily on days 1, 2, 3 and 4 after infection.

The mice were observed for mortality over a 14-day period following infection and the efficacy of each drug was evaluated in terms of survival rate (%).

The results are set forth in the following table.

| | Dose (mg/kg) | Survival rate (%) |
|---|---|---|
| Compound (A) | 0.1<br>0.01 | 70<br>80 |
| Acyclovir | 15<br>5 | 50<br>0 |
| Control | 0 | 0 |

The pharmaceutical composition of this invention for Cytomegalovirus infection can be supplied in various solid, semisolid and liquid forms as prepared by formulating the active ingredient of the invention with an organic or inorganic vehicle or excipient suited for oral or parental administration. Thus, the active ingredient is generally mixed with nontoxic, pharmaceutically acceptable vehicles to provide tablets, pellets, capsules, suppositories, injection, solutions, emulsions, suspensions and other dosage forms. The vehicles mentioned above are those which can be used in the manufacture of a solid, semisolid or liquid preparation. Thus, the vehicle includes, among others, water, glucose, lactose, gum arabic, gelatin, mannitol, starch paste, magnesium trisilicate, talc, methylcellulose, polyethylene glycol, corn starch, keratin, colloidal silica, potato starch, urea and so on. In addition, various auxiliary agents, stabilizers, thickeners, colorants,

perfumes, etc. can be incorporated. This pharmaceutical composition for Cytomegalovirus infection may contain a suitable preservative or bacteriostatic agent for sustained stability of the active ingredient. The amount of the active ingredient in the pharmaceutical composition of this invention should be sufficient to insure a sufficient prophylactic effect or a desired therapeutic effect according to the stage and status of the disease.

For administration of this pharmaceutical composition for Cytomegalovirus infection to man or animals, it is preferably administered by intravenous, subcutaneous or intramuscular injection or orally. The dosage of the active ingredient of this invention, namely the compound (I) or a pharmaceutically acceptable salt thereof, depends on the patient's age, the severity of the disease and other conditions. Generally, however, a daily dose of about 0.01-10 mg as the active ingredient per kg body weight is administered to man or animals for purposes of prophylaxis or therapy.

Example 1

| (Tablet) | |
|---|---|
| Compound (A) | 200 mg |
| Mannitol | 400 mg |
| Magnesium stearate | 10 mg |
| Starch | 50 mg |

Using the above components, tablets are manufactured by the established pharmaceutical procedure.

Example 2

| (Capsule) | |
|---|---|
| Compound (A) | 300 mg |
| Magnesium stearate | 15 mg |

Using the above components, capsules are manufactured by the established pharmaceutical procedure.

Example 3

(Injection)

Compound (A), 100 mg, is aseptically filled into a vial and sealed free of moisture and bacteria. To each vial is added 2 ml of lidocain 0.5% injection extemporaneously to prepare an injection.

**Claims**

**1.** A pharmaceutical composition for infectious diseases due to Cytomegalovirus comprising an effective amount of a compound of the formula:

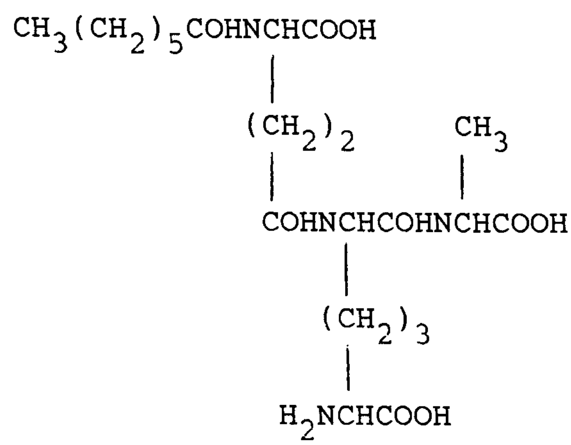

or a pharmaceutically acceptable salt thereof in admixture with vehicles.

**2.** A pharmaceutical composition according to claim 1 comprising an effective amount of a compound of the formula:

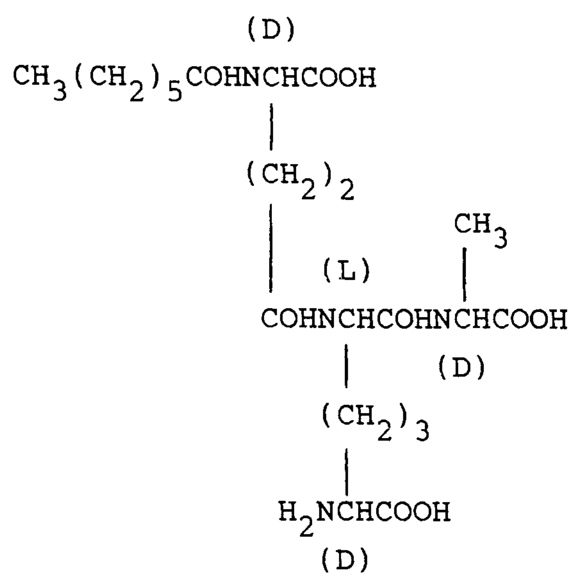

**3.** Use of a compound of the formula

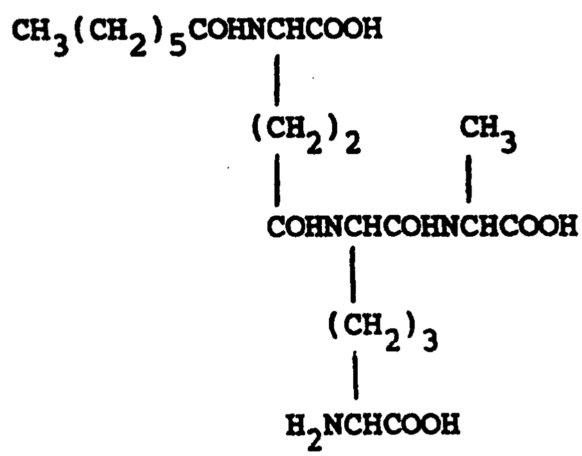

or a pharmaceutically acceptable salt thereof for the preparation of a medicament for preventing or treating infectious diseases due to Cytomegalovirus.